# EUROPÄISCHE PATENTANMELDUNG

(11) **EP 1 902 618 A1**
(43) Veröffentlichungstag der Anmeldung: **26.03.2008**
(21) Anmeldenummer: 06017221.0
(22) Anmeldetag: 17.08.2006
(51) Int. Cl.: A01N 47/36, A01N 25/12, B01J 2/02, B01J 2/04

(54) **Verfahren zur Herstellung von Sulfonamidsalzen**

(71) Anmelder: Bayer CropScience AG, 40789 Monheim (DE)
(72) Erfinder: Schnabel, Gerhard., 63820 Elsenfeld (DE); Haase, Detlef., 65929 Frankfurt (DE); Deckwer, Roland., 65929 Frankfurt (DE); Krause, Hans-Peter., 65719 Hofheim (DE); Bommel, Martin., 65843 Sulzbach (DE)

(57) **Zusammenfassung**

Verfahren zur Herstellung von Sulfonamidsalzen, enthaltend die Schritte
a) Umsetzung von einem oder mehreren Sulfonamiden mit einer oder mehreren Basen in einem oder mehreren Lösungsmitteln zu Sulfonamidsalzen,
b) Trocknung des in Schritt a) erhaltenen Produkts durch Versprühen in einer Trocknungsvorrichtung.

## Beschreibung

Die vorliegende Erfindung betrifft das technische Gebiet der Herstellverfahren von Pflanzenschutzmittelwirkstoffen sowie deren Formulierungen, insbesondere die Herstellung von agrochemisch wirksamen Sulfonamidsalzen und deren Formulierungen.

Verfahren zur Herstellung von Salzen von agrochemisch wirksamen Sulfonamiden sowie deren Formulierung sind dem Fachmann gut bekannt (z.B. "Chemistry of Sulfonylurea Herbicides", James V. Hay, Pesticide Science, 29 (1990) 3, 247 ff., WO 96/15119, DE 199 633 83 A1, DE 199 633 95 A1, DE 199 030 64 A1). Allerdings können bei der Salzbildung von Sulfonamiden Probleme auftreten wie unerwünschte Nebenreaktionen, z.B. die Verseifung von Estergruppen oder die Spaltung der Sulfonamidbrücke durch Hydrolyse oder Alkoholyse. Probleme kann es auch bei der Aufarbeitung der Sulfonamidsalze geben, z.B. beim Entfernen von Lösungsmittel.

Die Aufgabe der vorliegenden Erfindung bestand darin, ein Verfahren zur Verfügung zu stellen, womit Sulfonamidsalze in guter Qualität und mit hohen Ausbeuten herstellbar sind.

Überraschenderweise wurde nun gefunden, dass diese Aufgabe durch das Verfahren der vorliegenden Erfindung gelöst werden kann.

Gegenstand der vorliegenden Erfindung ist somit ein Verfahren zur Herstellung von Sulfonamidsalzen, enthaltend die Schritte
a) Umsetzung von einem oder mehreren Sulfonamiden mit einer oder mehreren Basen in einem oder mehreren Lösungsmitteln zu Sulfonamidsalzen,
b) Trocknung des in Schritt a) erhaltenen Produkts durch Versprühen in einer Trocknungsvorrichtung.

Dabei wird im allgemeinen zu einem Gemisch von Sulfonamid und Lösungsmittel Base (in der Regel ein Äquivalent) zugesetzt und das resultierende Produkt anschließend getrocknet (z.B. durch Sprühtrocknung oder Wirbelschichttrocknung), wobei das Sulfonamidsalz in fester Form anfällt. Optional kann das in Schritt a) erhaltene Produkt vor der Trocknung b) (z.B. durch Sprühtrocknung oder Wirbelschichttrocknung) einem Reinigungsschritt (z.B. Filtration oder Extraktion) und/oder einem Homogenisierungsschritt (z.B. Durchmischung mittels Rührer oder Rotor-Stator-Mischer) unterzogen werden.

Unter dem Begriff Sulfonamid wird im Rahmen dieser Beschreibung jeweils die Ausgangsverbindung, insbesondere eine Neutralverbindung verstanden, die durch Umsetzung mit einer geeigneten Base in das entsprechende Sulfonamidsalz überführt werden kann.

Als Sulfonamide bevorzugt sind Phenylsulfonamide, Heteroarylsulfonamide und Sulfondiamide wie Cyclosulfamuron oder (Alkylsulfonyl)alkylamino-sulfonamide z.B. Amidosulfuron. Als Phenylsulfonamide a) kommen beispielsweise Verbindungen aus der Gruppe der Phenylsulfonylaminocarbonyltriazolinone oder der Phenylsulfonylharnstoffe, bevorzugt aus der Gruppe der Phenylsulfonylharnstoffe in Frage. Unter dem Begriff Phenylsulfonylharnstoffe werden auch solche Sulfonylharnstoffe verstanden, bei denen die Phenylgruppe über einen Spacer wie CH₂, O oder NH an die Sulfonylgruppe (SO₂) gebunden ist. Beispiele für Phenylsulfonylaminocarbonyltriazolinone sind Flucarbazone oder Propoxycarbazone. Die Sulfonamide sind kommerziell erhältlich und/oder nach bekannten Verfahren herstellbar wie sie z.B. beschrieben sind in EP-A-7687, EP-A-30138, US 5,057,144 und US 5,534,486.

Als Phenylsulfonamide kommen beispielsweise Phenylsulfonamide der allgemeinen Formel (I) in Frage,

R^{α}-(A)ₘ-SO₂-NR^{β}-CO-(NR^{γ})ₙ - R^{δ} (I)

worin
- R^{α}: ein Phenylrest ist, der unsubstituiert oder substituiert ist, und wobei der Phenylrest inklusive Substituenten 1-30 C-Atome, vorzugsweise 1-20 C-Atome aufweist,
- R^{β}: ein Wasserstoffatom oder ein Kohlenwasserstoffrest ist, der unsubstituiert oder substituiert ist und inklusive Substituenten 1-10 C-Atome aufweist, z.B. unsubstituiertes oder substituiertes C₁-C₆-Alkyl, vorzugsweise ein Wasserstoffatom oder Methyl,
- R^{γ}: ein Wasserstoffatom oder ein Kohlenwasserstoffrest ist, der unsubstituiert oder substituiert ist und inklusive Substituenten 1-10 C-Atome aufweist, z.B. unsubstituiertes oder substituiertes C₁-C₆-Alkyl, vorzugsweise ein Wasserstoffatom oder Methyl,
- A: gleich CH₂, O oder NH, vorzugsweise O, ist,
- m: gleich Null oder 1,
- n: gleich Null oder 1, vorzugsweise gleich 1, und
- R^{δ}: ein heterocyclischer Rest wie ein Pyrimidinylrest, ein Triazinylrest oder ein Triazolinonrest ist.

Bevorzugte Phenylsulfonamide sind Phenylsulfonylharnstoffe, beispielsweise Phenylsulfonylharnstoffe der allgemeinen Formel (II),

R^{α}-(A)ₘ-SO₂-NR^{β}-CO-NR^{γ}- R^{δ} (II)

worin
- R^{α}: ein Phenylrest ist, der unsubstituiert oder substituiert ist, und wobei der Phenylrest inklusive Substituenten 6-30 C-Atome, vorzugsweise 6-20 C-Atome aufweist,
- R^{β}: ein Wasserstoffatom oder ein Kohlenwasserstoffrest ist, der unsubstituiert oder substituiert ist und inklusive Substituenten 1-10 C-Atome aufweist, z.B. unsubstituiertes oder substituiertes C₁-C₆-Alkyl, vorzugsweise ein Wasserstoffatom oder Methyl,
- R^{γ}: ein Wasserstoffatom oder ein Kohlenwasserstoffrest ist, der unsubstituiert oder substituiert ist und inklusive Substituenten 1-10 C-Atome aufweist, z.B. unsubstituiertes oder substituiertes C₁-C₆-Alkyl, vorzugsweise ein Wasserstoffatom oder Methyl,
- A: gleich CH₂, O oder NH, vorzugsweise O, ist,
- m: gleich Null oder 1, und
- R^{δ}: ein heterocyclischer Rest wie ein Pyrimidinylrest oder ein Triazinylrest ist.

Bevorzugt sind Phenylsulfonylharnstoffe der Formel (III), 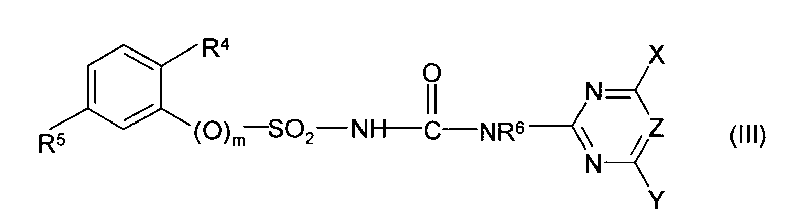 worin
- R⁴: C₁-C₄-Alkoxy, vorzugsweise C₂-C₄-Alkoxy, oder CO-R^{a} ist, worin R^{a} gleich OH, C₁-C₄-Alkoxy oder NR^{b}R^{c} ist, worin R^{b} und R^{c} unabhängig voneinander gleich oder verschieden H oder C₁-C₄-Alkyl sind,
- R⁵: Halogen, vorzugsweise lod, oder (A)ₙ-NR^{d}R^{e} ist, worin n gleich Null oder 1 ist, A eine Gruppe CR'R" ist, worin R' und R" unabhängig voneinander gleich oder verschieden H oder C₁-C₄-Alkyl sind, R^{d} gleich H oder C₁-C₄-Alkyl ist und R^{e} ein Acylrest wie Formyl oder C₁-C₄-Alkyl-Sulfonyl ist, und R⁵ für den Fall, daß R⁴ gleich C₁-C₄-Alkoxy, vorzugsweise C₂-C₄-Alkoxy, bedeutet auch H sein kann,
- R⁶: H oder C₁-C₄-Alkyl ist,
- m: gleich Null oder 1 ist,
- X und Y: unabhängig voneinander gleich oder verschieden Halogen oder NR'R", worin R' und R" gleich oder verschieden H oder C₁-C₄-Alkyl sind, oder C₁-C₆-Alkyl, C₁-C₆-Alkoxy, C₁-C₆-Alkylthio, C₃-C₆-Cycloalkyl, C₂-C₆-Alkenyl, C₂-C₆-Alkynyl, C₃-C₆-Alkenyloxy oder C₃-C₆-Alkynyloxy sind, wobei jeder der acht letztgenannten Reste unsubstituiert oder durch einen oder mehrere Reste aus der Gruppe Halogen, C₁-C₄-Alkoxy und C₁-C₄-Alkylthio substituiert ist, vorzugsweise C₁-C₄-Alkyl oder C₁-C₄-Alkoxy, und
- Z: gleich CH oder N ist.

Besonders bevorzugt sind Phenylsulfonylharnstoffe der allgemeinen Formel (lll), worin
a) R⁴ gleich CO-(C₁-C₄-Alkoxy) ist, R⁵ gleich Halogen, vorzugsweise Jod ist, oder R⁵ gleich CH₂-NHR^{e} ist, worin R^{e} ein Acylrest, vorzugsweise C₁-C₄-Alkyl-Sulfonyl ist, und m gleich Null ist,
b) R⁴ gleich CO-N(C₁-C₄-Alkyl)₂, R⁵ gleich NHR^{e} ist, worin R^{e} ein Acylrest, vorzugsweise Formyl ist, und m gleich Null ist, oder
c) R⁴ gleich C₂-C₄-Alkoxy, R⁵ gleich H und m gleich 1 ist.

Typische Phenylsulfonylharnstoffe sind unter anderem die nachfolgend aufgeführten Verbindungen: Bensulfuron-methyl, Chlorimuron-Ethyl, Chlorsulfuron, Cinosulfuron, Cyclosulfamuron, Ethametsulfuron-methyl, Ethoxysulfuron, Metsulfuron-methyl, Oxasulfuron, Primisulfuron-methyl, Prosulfuron, Sulfometuron-methyl, Triasulfuron, Tribenuron-methyl, Triflusulfuron-methyl, Tritosulfuron, lodosulfuron-methyl (WO 92/13845), Mesosulfuron-methyl (Agrow Nr. 347, 3. März 2000, Seite 22 (PJB Publications Ltd. 2000)) und Foramsulfuron (Agrow Nr. 338, 15. Oktober 1999, Seite 26 (PJB Publications Ltd. 1999)).

Besonders bevorzugte Phenylsulfonamide sind: lodosulfuron-methyl (A1), Mesosulfuron-methyl (A2), Foramsulfuron (A3), Flucarbazone (A4), Propoxycarbazone (A5) und Ethoxysulfuron (A6), Metsulfuron-methyl (A7), Tribenuron-methyl (A8), Chlorsulfuron (A9).

Als Heteroarylsulfonamide a) kommen beispielsweise Verbindungen aus der Gruppe der Heteroarylsulfonylaminocarbonyltriazolinone oder der Heteroarylsulfonylharnstoffe, bevorzugt aus der Gruppe der Heteroarylsulfonylharnstoffe in Frage. Unter dem Begriff Heteroarylsulfonylharnstoffe werden auch solche Sulfonylharnstoffe verstanden, bei denen die Heteroarylgruppe über einen Spacer wie CH₂, O oder NH an die Sulfongruppe (SO₂) gebunden ist.

Als Heteroarylsulfonamide kommen beispielsweise Sulfonamide der allgemeinen Formel (IV) in Frage,

R^{α'}-(A')_{m'}-SO₂-NR^{β'}-CO-(NR^{γ'})_{n'} - R^{δ'} (IV)

worin
- R^{α}': ein Heteroarylrest ist, der unsubstituiert oder substituiert ist, und wobei der Heteroarylrest inklusive Substituenten 1-30 C-Atome, vorzugsweise 2-20 C-Atome aufweist,
- R^{β}': ein Wasserstoffatom oder ein Kohlenwasserstoffrest ist, der unsubstituiert oder substituiert ist und inklusive Substituenten 1-10 C-Atome aufweist, z.B. unsubstituiertes oder substituiertes C₁-C₆-Alkyl, vorzugsweise ein Wasserstoffatom oder Methyl,
- R^{γ}': ein Wasserstoffatom oder ein Kohlenwasserstoffrest ist, der unsubstituiert oder substituiert ist und inklusive Substituenten 1-10 C-Atome aufweist, z.B. unsubstituiertes oder substituiertes C₁-C₆-Alkyl, vorzugsweise ein Wasserstoffatom oder Methyl,
- A': gleich CH₂, O oder NH, vorzugsweise O, ist,
- m': gleich Null oder 1,
- n': gleich Null oder 1, vorzugsweise gleich 1, und
- R^{δ}': ein heterocyclischer Rest wie ein Pyrimidinylrest, ein Triazinylrest oder ein Triazolinonrest ist.

Bevorzugte Heteroarylsulfonamide sind Heteroarylsulfonylharnstoffe, beispielsweise Sulfonylharnstoffe der allgemeinen Formel (V),

R-^{α'}-(A')_{m'}-SO₂-NR^{β'}-CO-NR^{γ'}- R^{δ'} (V)

worin
- R^{α'}: ein Heteroarylrest ist, der unsubstituiert oder substituiert ist, und wobei der Heteroarylrest inklusive Substituenten 1-30 C-Atome, vorzugsweise 2-20 C-Atome aufweist,
- R^{β}': ein Wasserstoffatom oder ein Kohlenwasserstoffrest ist, der unsubstituiert oder substituiert ist und inklusive Substituenten 1-10 C-Atome aufweist, z.B. unsubstituiertes oder substituiertes C₁-C₆-Alkyl, vorzugsweise ein Wasserstoffatom oder Methyl,
- R^{γ}': ein Wasserstoffatom oder ein Kohlenwasserstoffrest ist, der unsubstituiert oder substituiert ist und inklusive Substituenten 1-10 C-Atome aufweist, z.B. unsubstituiertes oder substituiertes C₁-C₆-Alkyl, vorzugsweise ein Wasserstoffatom oder Methyl,
- A': gleich CH₂, O oder NH, vorzugsweise O, ist,
- m': gleich Null oder 1, und
- R^{δ}': ein heterocyclischer Rest wie ein Pyrimidinylrest oder ein Triazinylrest ist.

Besonders bevorzugt sind Heteroarylsulfonamide der nachfolgend genannten Formel (VI),

R^{α'}-SO₂-NH-CO-(NR^{γ'})_{n'}-R^{δ'} (VI)

,worin
- R^{α}': ein substituierter Heteroarylrest, wie substituiertes Pyridyl, Thienyl, Pyrazolyl oder lmidazolyl,
- R^{γ}': H, (C₁-C₃)Alkyl, optional substituiert mit Halogen (F, C, Br, I) oder (Halo)Alkoxy (C₁-C₃), bevorzugt H oder Methyl,
- für n': gleich 1, R^{δ}' ein Pyrimidinylrest oder ein Triazinylrest ist, vorzugsweise 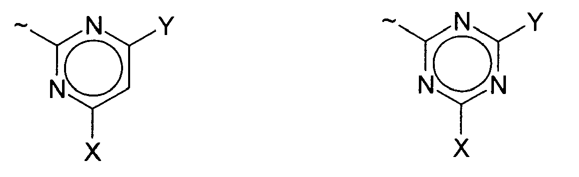
und für n' gleich Null, R^{δ}' ein Triazolinonrest ist, vorzugsweise 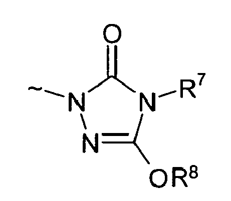
- R⁷: (C₁-C₁₀)Alkyl, welches optional substituiert ist mit Halogen (F, Cl, Br, I) oder (C₁-C₃)Halo-Alkyl,
- R⁸: (C₁-C₁₀)Alkyl, welches optional substituiert ist mit Halogen (F, Cl, Br, I) oder (C₁-C₃)Halo-Alkyl,
- X und Y: unabhängig voneinander gleich oder verschieden Halogen oder NR'R", worin R' und R" gleich oder verschieden H oder C₁-C₄-Alkyl sind, oder C₁-C₆-Alkyl, C₁-C₆-Alkoxy, C₁-C₆-Alkylthio, C₃-C₆-Cycloalkyl, C₂-C₆-Alkenyl, C₂-C₆-Alkynyl, C₃-C₆-Alkenyloxy oder C₃-C₆-Alkynyloxy sind, wobei jeder der acht letztgenannten Reste unsubstituiert oder durch einen oder mehrere Reste aus der Gruppe Halogen, C₁-C₄-Alkoxy und C₁-C₄-Alkylthio substituiert ist, vorzugsweise C₁-C₄-Alkyl oder C₁-C₄-Alkoxy.

Besonders bevorzugt ist R^{α}' gleich 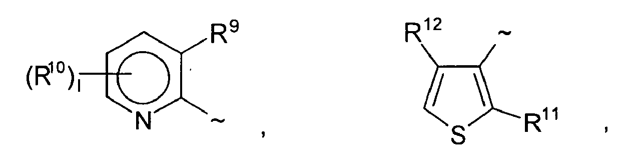 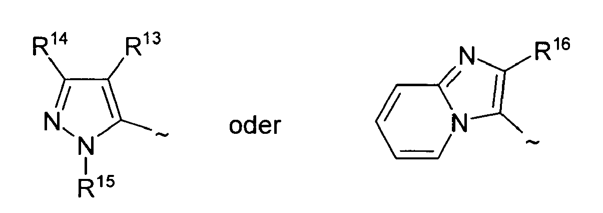 , worin
- R⁹: (C₁-C₆)Alkyl, (C₁-C₆)Alkoxy, (C₂-C₆)Alkenyloxy, (C₂-C₆)Alkinyloxy, (C₁-C₆)Alkylsulfonyl, (C₁-C₆)Alkylcarbonyl, (C₁-C₆)Alkoxycarbonyl, (C₂-C₆)Alkenyloxycarbonyl, (C₂-C₆)Alkinyloxycarbonyl, CONR'R", (C₁-C₆)Halo-Alkyl, (C₁-C₆)Halo-Alkoxy, (C₂-C₆)Halo-Alkenyloxy, (C₂-C₆)Halo-Alkinyloxy, (C₁-C₆)Halo-Alkylsulfonyl, (C₁-C₆)Halo-Alkylcarbonyl, (C₁-C₆)Halo-Alkoxycarbonyl, (C₂-C₆) Halo-Alkenyloxycarbonyl, (C₂-C₆)Halo-Alkinyloxycarbonyl oder unsubstituiertes oder substituiertes Dioxazinyl,
- R¹⁰: H, (C₁-C₃)Alkyl, (C₁-C₃)Alkoxy, (C₁-C₃)Halo-Alkyl, (C₁-C₃)Halo-Alkoxy oder Halogen (F, Cl, Br, I),
- I: Null oder 1,
- R¹¹: (C₁-C₆)Alkyl, (C₁-C₆)Alkoxy, (C₂-C₆)Alkenyloxy, (C₂-C₆)Alkinyloxy, (C₁-C₆)Alkylsulfonyl, (C₁-C₆)Alkylcarbonyl, (C₁-C₆)Alkoxycarbonyl, (C₂-C₆)Alkenyloxycarbonyl, (C₂-C₆)Alkinyloxycarbonyl, (C₁-C₆)Halo-Alkyl, (C₁-C₆)Halo-Alkoxy, (C₂-C₆)Halo-Alkenyloxy, (C₂-C₆)Halo-Alkinyloxy, (C₁-C₆)Halo-Alkylsulfonyl, (C₁-C₆)Halo-Alkylcarbonyl, (C₁-C₆)Halo-Alkoxycarbonyl, (C₂-C₆)Halo-Alkenyloxycarbonyl, (C₂-C₆)Halo-Alkinyloxycarbonyl, CONR'R",
- R¹²: Halogen (F, Cl, Br, l), (C₁-C₆)Alkyl, (C₁-C₆)Alkoxy, (C₁-C₆)Alkylsulfonyl, (C₁-C₆)Alkoxycarbonyl, (C₂-C₆)Alkenyloxycarbonyl, (C₂-C₆)Alkinyloxycarbonyl, (C₁-C₆)Halo-Alkyl, (C₁-C₆)Halo-Alkoxy, (C₁-C₆)Halo-Alkylsulfonyl, (C₁-C₆)Halo-Alkoxycarbonyl, (C₂-C₆)Halo-Alkenyloxycarbonyl, (C₂-C₆)Halo-Alkinyloxycarbonyl,
- R¹³: (C₁-C₆)Alkoxycarbonyl, (C₂-C₆)Alkenyloxycarbonyl, (C₂-C₆)Alkinyloxycarbonyl, (C₁-C₆)Alkyl, (C₁-C₆)Alkoxy, (C₁-C₆)Alkylsulfonyl, (C₁-C₆)Halo-Alkoxycarbonyl, (C₂-C₆)Halo-Alkenyloxycarbonyl, (C₂-C₆)Halo-Alkinyloxycarbonyl, (C₁-C₆)Halo-Alkyl, (C₁-C₆)Halo-Alkoxy, (C₁-C₆)Halo-Alkylsulfonyl, Halogen (F, Cl, Br, I), CONR'R", oder R¹³ ist ein heterocylischer Ring der gesättigt, ungesättigt oder aromatisch sein kann und vorzugsweise 4-6-Ringatome und eines oder mehrere Heteroatome aus der Gruppe N, O, S enthält, und optional durch einen oder mehrere Substituenten, vorzugsweise aus der Gruppe (C₁-C₃)Alkyl, (C₁-C₃)Alkoxy, (C₁-C₃)Halo-Alkyl, (C₁-C₃)Halo-Alkoxy oder Halogen substituiert sein kann, besonders bevorzugt 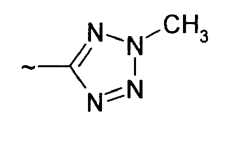
- R¹⁴: H, Halogen (F, C, Br, I), (C₁-C₆)Alkyl, (C₁-C₆)Halo-Alkyl,
- R¹⁵: H, (C₁-C₆)Alkyl, (C₁-C₆)Halo-Alkyl,
- R¹⁶: (C₁-C₆)Alkyl, (C₁-C₆)Alkoxy, (C₂-C₆)Alkenyloxy, (C₂-C₆)Alkinyloxy, (C₁-C₆)Alkylsulfonyl, (C₁-C₆)Alkylcarbonyl, (C₁-C₆)Alkoxycarbonyl, (C₂-C₆)Alkenyloxycarbonyl, (C₂-C₆)Alkinyloxycarbonyl, (C₁-C₆)Halo-Alkyl, (C₁-C₆)Halo-Alkoxy, (C₂-C₆)Halo-Alkenyloxy, (C₂-C₆)Halo-Alkinyloxy, (C₁-C₆)Halo-Alkylsulfonyl, (C₁-C₆)Halo-Alkylcarbonyl, (C₁-C₆)Halo-Alkoxycarbonyl, (C₂-C₆)Halo-Alkenyloxycarbonyl, (C₂-C₆)Halo-Alkinyloxycarbonyl, CONR'R", insbesondere SO₂Ethyl, und
- R' und R": unabhängig voneinander H, (C₁-C₆)Alkyl, (C₁-C₆)Haloalkyl, (C₂-C₆)Alkenyl, (C₂-C₆)Haloalkenyl, (C₂-C₆)Alkinyl, (C₂-C₆)Haloalkinyl sind, oder NR'R" bildet einen heterocylischen Ring der gesättigt, ungesättigt oder aromatisch sein kann und vorzugsweise 4-6-Ringatome und eines oder mehrere Heteroatome aus der Gruppe N, O, S enthält, und optional durch einen oder mehrere Substituenten, vorzugsweise aus der Gruppe (C₁-C₃)Alkyl, (C₁-C₃)Alkoxy, (C₁-C₃)Halo-Alkyl, (C₁-C₃)Halo-Alkoxy oder Halogen substituiert sein kann.

Besonders bevorzugte Heteroarylsulfonylharnstoffe sind z.B. Nicosulfuron (A10), Rimsulfuron (A11), Thifensulfuron-Methyl (A12), Pyrazosulfuron-Ethyl (A13), Flupyrsulfuron-Methyl (A14), Sulfosulfuron (A15), Trifloxysulfuron (A16), Azimsulfuron (A17), Flazasulfuron (A18) und Flucetosulfuron (1-[3-[[[[(4,6-dimethoxy-2-pyrimidinyl)amino]carbonyl]amino]sulfonyl]-2-pyridinyl]-2-fluoropropyl methoxyacetat, (A19)) oder Dioxazinyl-Pyridylsulfonylharnstoffe, z.B. solche der allgemeinen Formel (VII) 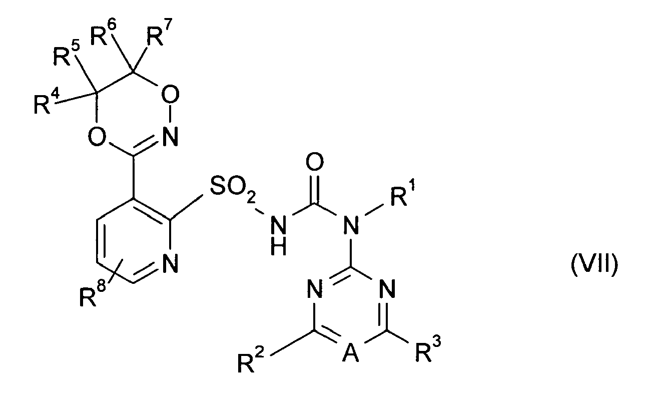 worin
- A: für Stickstoff oder eine CR¹¹-Gruppierung steht, wobei
R¹¹ für Wasserstoff, Alkyl, Halogen und Haloalkyl steht,
- R¹: für Wasserstoff oder einen gegebenenfalls substituierten Rest aus der Reihe Alkyl, Alkoxy, Alkoxyalkyl, Alkenyl, Alkinyl, Cycloalkyl, Cycloalkylalkyl, Aralkyl und Aryl steht,
- R²: für Wasserstoff, Halogen oder für jeweils gegebenenfalls durch Halogen substituiertes Alkyl, Alkoxy, Alkylthio, Alkylamino oder Dialkylamino mit jeweils 1 bis 6 Kohlenstoffatomen steht,
- R³: für Wasserstoff, Halogen oder für jeweils gegebenenfalls durch Halogen substituiertes Alkyl, Alkoxy, Alkylthio, Alkylamino oder Dialkylamino mit jeweils 1 bis 6 Kohlenstoffatomen steht,
- R⁴ - R⁷: unabhängig voneinander für Wasserstoff, Halogen, Cyano, Thiocyanato oder für jeweils gegebenenfalls durch Halogen substituiertes Alkyl, Alkoxy, Alkylthio, Alkylsulfinyl, Alkylsulfonyl, Alkylamino, Alkylcarbonyl, Alkoxycarbonyl, Alkylaminocarbonyl mit jeweils 1 bis 3 Kohlenstoffatomen stehen,
- R⁸: für Wasserstoff, Halogen, Cyano, Thiocyanato oder für jeweils gegebenenfalls durch Halogen substituiertes Alkyl, Alkoxy, Alkylthio, Alkylsulfinyl, Alkylsulfonyl, Alkylamino, Alkylcarbonyl, Alkoxycarbonyl, Alkylaminocarbonyl mit jeweils 1 bis 3 Kohlenstoffatomen steht,
wobei in den vorgenannten Resten die Alkyl- und Alkylengruppen jeweils 1 bis 6 C-Atome, die Alkenyl- und Alkinylgruppen jeweils 2 bis 6 C-Atome, die Cycloalkylgruppen jeweils 3 bis 6 C-Atome und die Arylgruppen jeweils 6 bzw. 10 C-Atome enthalten können.

Dioxazinyl-Pyridylsulfonylharnstoffe wie die Verbindungen der Formel (A) sind bekannt, ebenso wie deren Herstellung, z.B. aus US 5,476,936 deren Inhalt hiermit in die vorliegende Beschreibung aufgenommen wird.

Bevorzugt sind Verbindungen der Formel (VII),
worin
- A: für Stickstoff oder eine CH-Gruppierung steht,
- R¹: für Wasserstoff oder einen gegebenenfalls durch Halogen substituierten Rest aus der Reihe Alkyl, Alkoxy, Alkoxyalkyl, Alkenyl und Alkinyl mit jeweils bis zu 3 Kohlenstoffatomen steht,
- R²: für Wasserstoff, Halogen oder für jeweils gegebenenfalls durch Halogen substituiertes Alkyl, Alkoxy, Alkylthio, Alkylamino oder Dialkylamino mit jeweils 1 bis 3 Kohlenstoffatomen in den Alkylresten steht,
- R³: für Wasserstoff, Halogen oder für jeweils gegebenenfalls durch Halogen substituiertes Alkyl, Alkoxy, Alkylthio, Alkylamino oder Dialkylamino mit jeweils 1 bis 3 Kohlenstoffatomen in den Alkylresten steht,
- R⁴ - R⁷: unabhängig voneinander für Wasserstoff, Halogen, Cyano, Thiocyanato oder für jeweils gegebenenfalls durch Halogen substituiertes Alkyl, Alkoxy, Alkylthio, Alkylsulfinyl, Alkylsulfonyl, Alkylamino, Alkylcarbonyl, Alkoxycarbonyl oder Alkylaminocarbonyl mit jeweils 1 bis 3 Kohlenstoffatomen in den Alkylresten steht,
- R⁸: für Wasserstoff, Halogen, Cyano, Thiocyanato oder für jeweils gegebenenfalls durch Halogen substituiertes Alkyl, Alkoxy, Alkylthio, Alkylsulfinyl, Alkylsulfonyl, Alkylamino, Alkylcarbonyl, Alkoxycarbonyl oder Alkylaminocarbonyl mit jeweils 1 bis 3 Kohlenstoffatomen in den Alkylresten steht.

Insbesondere bevorzugt sind Verbindungen der Formel (VII),
worin
- A: für Stickstoff oder eine CH-Gruppierung steht,
- R¹: für Wasserstoff, Methyl, Ethyl, Methoxy, Methoxymethyl oder Ethoxy steht,
- R²: für Wasserstoff, Chlor, Methyl, Ethyl, Trifluoromethyl, Methoxy, Ethoxy, Difluoromethoxy, Methylthio, Methylamino oder Dimethylamino steht,
- R³: für Wasserstoff, Chlor, Methyl, Ethyl, Trifluoromethyl, Methoxy, Ethoxy, Difluoromethoxy, Methylthio, Methylamino oder Dimethylamino steht,
- R⁴ - R⁷: unabhängig voneinander für Wasserstoff, Fluor, Chlor, Cyano, oder für jeweils gegebenenfalls durch Chlor oder Fluor substituiertes Methyl, Methylthio, Methylsulfinyl, Methylsulfonyl, Methoxycarbonyl und Ethoxycarbonyl steht, vorzugsweise für Wasserstoff,
- R⁸: für Wasserstoff, Fluor, Chlor, Brom, Cyano oder für jeweils gegebenenfalls durch Chlor oder Fluor substituiertes Methyl, Methoxy, Ethoxy, Methylthio, Ethylthio, Methylsulfinyl, Ethylsulfinyl, Methylsulfonyl, Ethylsulfonyl, Methyl-oder Dimethylamino steht, vorzugsweise für Wasserstoff.

Besonders bevorzugt sind Verbindungen der Formel (VII),
worin
- A: für Stickstoff steht,
- R¹: für Wasserstoff oder Methyl steht,
- R²: für Wasserstoff, Chlor, Methyl, Ethyl, Trifluoromethyl, Methoxy, Ethoxy, Difluoromethoxy, Methylthio, Methylamino oder Dimethylamino steht,
- R³: für Wasserstoff, Chlor, Methyl, Ethyl, Trifluoromethyl, Methoxy, Ethoxy, Difluoromethoxy, Methylthio, Methylamino oder Dimethylamino steht,
- R⁴ - R⁷: für Wasserstoff steht,
- R⁸: für Wasserstoff steht.

Ebenfalls besonders bevorzugt sind Verbindungen der Formel (VII),
worin
- A: für eine CH-Gruppierung steht,
- R¹: für Wasserstoff oder Methyl steht,
- R²: für Wasserstoff, Chlor, Methyl, Ethyl, Trifluoromethyl, Methoxy, Ethoxy, Difluoromethoxy, Methylthio, Methylamino oder Dimethylamino steht,
- R³: für Wasserstoff, Chlor, Methyl, Ethyl, Trifluoromethyl, Methoxy, Ethoxy, Difluoromethoxy, Methylthio, Methylamino oder Dimethylamino steht,
- R⁴ - R⁷: für Wasserstoff steht,
- R⁸: für Wasserstoff steht.

Die oben aufgeführten allgemeinen oder in Vorzugsbereichen aufgeführten Restedefinitionen können untereinander, also auch zwischen den angegebenen bevorzugten Bereichen beliebig kombiniert werden.

Besonders bevorzugte Dioxazinyl-Pyridylsulfonylharnstoffe der Formel (VII) sind in der nachfolgenden Tabelle A genannt.

**Tabelle A: Beispiele für Verbindungen der Formel (VII) mit R⁴ = R⁵ = R⁶ = R⁷ = R⁸ = H:**

| Bsp.-Nr. | R¹ | A | R² | R³ |
|---|---|---|---|---|
| A20 | H | CH | OCH₃ | OC₂H₅ |
| A21 | H | CH | OCH₃ | CH₃ |
| A22 | H | CH | OCH₃ | C₂H₅ |
| A23 | H | CH | OCH₃ | CF₃ |
| A24 | H | CH | OCH₃ | OCF₂H |
| A25 | H | CH | OCH₃ | NHCH₃ |
| A26 | H | CH | OCH₃ | N(CH₃)₂ |
| A27 | H | CH | OCH₃ | Cl |
| A28 | H | CH | OCH₃ | OCH₃ |
| A29 | H | CH | OC₂H₅ | OC₂H₅ |
| A30 | H | CH | OC₂H₅ | CH₃ |
| A31 | H | CH | OC₂H₅ | C₂H₅ |
| A32 | H | CH | OC₂H₅ | CF₃ |
| A33 | H | CH | OC₂H₅ | OCF₂H |
| A34 | H | CH | OC₂H₅ | NHCH₃ |
| A35 | H | CH | OC₂H₅ | N(CH₃)₂ |
| A36 | H | CH | OC₂H₅ | Cl |
| A37 | H | CH | CH₃ | CH₃ |
| A38 | H | CH | CH₃ | C₂H₅ |
| A39 | H | CH | CH₃ | CF₃ |
| A40 | H | CH | CH₃ | OCF₂H |
| A41 | H | CH | CH₃ | NHCH₃ |
| A42 | H | CH | CH₃ | N(CH₃)₂ |
| A43 | H | CH | CH₃ | Cl |
| A44 | H | CH | C₂H₅ | C₂H₅ |
| A45 | H | CH | C₂H₅ | CF₃ |
| A46 | H | CH | C₂H₅ | OCF₂H |
| A47 | H | CH | C₂H₅ | NHCH₃ |
| A48 | H | CH | C₂H₅ | Cl |
| A49 | H | CH | CF₃ | CF₃ |
| A50 | H | CH | CF₃ | OCF₂H |
| A51 | H | CH | CF₃ | NHCH₃ |
| A52 | H | CH | CF₃ | N(CH₃)₂ |
| A53 | H | CH | CF₃ | Cl |
| A54 | H | CH | OCF₂H | OCF₂H |
| A55 | H | CH | OCF₂H | NHCH₃ |
| A56 | H | CH | OCF₂H | N(CH₃)₂ |
| A57 | H | CH | OCF₂H | Cl |
| A58 | H | CH | NHCH₃ | NHCH₃ |
| A59 | H | CH | NHCH₃ | N(CH₃)₂ |
| A60 | H | CH | NHCH₃ | Cl |
| A61 | H | CH | N(CH₃)₂ | N(CH₃)₂ |
| A62 | H | CH | N(CH₃)₂ | Cl |
| A63 | H | CH | Cl | Cl |
| A64 | H | N | OCH₃ | OCH₃ |
| A65 | H | N | OCH₃ | OC₂H₅ |
| A66 | H | N | OCH₃ | CH₃ |
| A67 | H | N | OCH₃ | C₂H₅ |
| A68 | H | N | OCH₃ | CF₃ |
| A69 | H | N | OCH₃ | OCF₂H |
| A70 | H | N | OCH₃ | NHCH₃ |
| A71 | H | N | OCH₃ | N(CH₃)₂ |
| A72 | H | N | OCH₃ | Cl |
| A73 | H | N | OC₂H₅ | OC₂H₅ |
| A74 | H | N | OC₂H₅ | CH₃ |
| A75 | H | N | OC₂H₅ | C₂H₅ |
| A76 | H | N | OC₂H₅ | CF₃ |
| A77 | H | N | OC₂H₅ | OCF₂H |
| A78 | H | N | OC₂H₅ | NHCH₃ |
| A79 | H | N | OC₂H₅ | N(CH₃)₂ |
| A80 | H | N | OC₂H₅ | Cl |
| A81 | H | N | CH₃ | CH₃ |
| A82 | H | N | CH₃ | C₂H₅ |
| A83 | H | N | CH₃ | CF₃ |
| A84 | H | N | CH₃ | OCF₂H |
| A85 | H | N | CH₃ | NHCH₃ |
| A86 | H | N | CH₃ | N(CH₃)₂ |
| A87 | H | N | CH₃ | Cl |
| A88 | H | N | C₂H₅ | C₂H₅ |
| A89 | H | N | C₂H₅ | CF₃ |
| A90 | H | N | C₂H₅ | OCF₂H |
| A91 | H | N | C₂H₅ | NHCH₃ |
| A92 | H | N | C₂H₅ | Cl |
| A93 | H | N | CF₃ | CF₃ |
| A94 | H | N | CF₃ | OCF₂H |
| A95 | H | N | CF₃ | NHCH₃ |
| A96 | H | N | CF₃ | N(CH₃)₂ |
| A97 | H | N | CF₃ | Cl |
| A98 | H | N | OCF₂H | OCF₂H |
| A99 | H | N | OCF₂H | NHCH₃ |
| A100 | H | N | OCF₂H | N(CH₃)₂ |
| A101 | H | N | OCF₂H | Cl |
| A102 | H | N | NHCH₃ | NHCH₃ |
| A103 | H | N | NHCH₃ | N(CH₃)₂ |
| A104 | H | N | NHCH₃ | Cl |
| A105 | H | N | N(CH₃)₂ | N(CH₃)₂ |
| A106 | H | N | N(CH₃)₂ | Cl |
| A107 | H | N | Cl | Cl |
| A108 | CH₃ | N | OCH₃ | OCH₃ |
| A109 | CH₃ | N | OCH₃ | OC₂H₅ |
| A110 | CH₃ | N | OCH₃ | CH₃ |
| A111 | CH₃ | N | OCH₃ | C₂H₅ |
| A112 | CH₃ | N | OCH₃ | CF₃ |
| A113 | CH₃ | N | OCH₃ | OCF₂H |
| A114 | CH₃ | N | OCH₃ | NHCH₃ |
| A115 | CH₃ | N | OCH₃ | N(CH₃)₂ |
| A116 | CH₃ | N | OCH₃ | Cl |
| A117 | CH₃ | N | OC₂H₅ | OC₂H₅ |
| A118 | CH₃ | N | OC₂H₅ | CH₃ |
| A119 | CH₃ | N | OC₂H₅ | C₂H₅ |
| A120 | CH₃ | N | OC₂H₅ | CF₃ |
| A121 | CH₃ | N | OC₂H₅ | OCF₂H |
| A122 | CH₃ | N | OC₂H₅ | NHCH₃ |
| A123 | CH₃ | N | OC₂H₅ | N(CH₃)₂ |
| A124 | CH₃ | N | OC₂H₅ | Cl |
| A125 | CH₃ | N | CH₃ | CH₃ |
| A126 | CH₃ | N | CH₃ | C₂H₅ |
| A127 | CH₃ | N | CH₃ | CF₃ |
| A128 | CH₃ | N | CH₃ | OCF₂H |
| A129 | CH₃ | N | CH₃ | NHCH₃ |
| A130 | CH₃ | N | CH₃ | N(CH₃)₂ |
| A131 | CH₃ | N | CH₃ | Cl |
| A132 | CH₃ | N | C₂H₅ | C₂H₅ |
| A133 | CH₃ | N | C₂H₅ | CF₃ |
| A134 | CH₃ | N | C₂H₅ | OCF₂H |
| A135 | CH₃ | N | C₂H₅ | NHCH₃ |
| A136 | CH₃ | N | C₂H₅ | Cl |
| A137 | CH₃ | N | CF₃ | CF₃ |
| A138 | CH₃ | N | CF₃ | OCF₂H |
| A139 | CH₃ | N | CF₃ | NHCH₃ |
| A140 | CH₃ | N | CF₃ | N(CH₃)₂ |
| A141 | CH₃ | N | CF₃ | Cl |
| A142 | CH₃ | N | OCF₂H | OCF₂H |
| A143 | CH₃ | N | OCF₂H | NHCH₃ |
| A144 | CH₃ | N | OCF₂H | N(CH₃)₂ |
| A145 | CH₃ | N | OCF₂H | Cl |
| A146 | CH₃ | N | NHCH₃ | NHCH₃ |
| A147 | CH₃ | N | NHCH₃ | N(CH₃)₂ |
| A148 | CH₃ | N | NHCH₃ | Cl |
| A149 | CH₃ | N | N(CH₃)₂ | N(CH₃)₂ |
| A150 | CH₃ | N | N(CH₃)₂ | Cl |
| A151 | CH₃ | N | Cl | Cl |
| A152 | H | N | N(CH₃)₂ | OCH₂CF₃ |
| A153 | H | CH | Cl | OCH₂CF₃ |
| A154 | H | CH | Cl | OCH₂CF₃ |

Besonders bevorzugte Heteroarylsulfonylaminocarbonyltriazolinone sind die nachfolgend genannten Verbindungen der Formel (VIII), die z.B. bekannt sind aus WO 03/026427, deren Inhalt hiermit in die vorliegende Beschreibung aufgenommen wird.

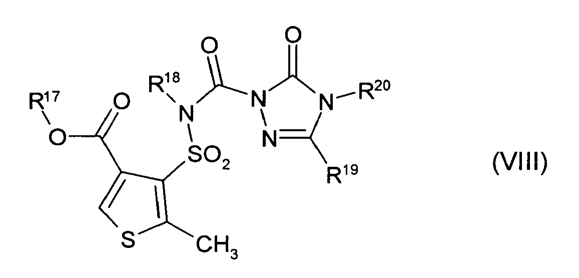

| Verbindung Nr. | R¹⁷ | R¹⁸ | R¹⁹ | R²⁰ |
|---|---|---|---|---|
| A155 | CH₃ | H | OC₂H₅ | CH₃ |
| A156 | CH₃ | H | OCH₃ | CH₃ |
| A157 | CH₃ | H | OC₃H₇-n | CH₃ |
| A158 | CH₃ | H | OC₃H₇-i | CH₃ |
| A159 | CH₃ | H | OCH₃ | cyclo-propyl |
| A160 | CH₃ | H | OC₂H₅ | cyclo-propyl |
| A161 | CH₃ | H | OC₃H₇-n | cyclo-propyl |
| A162 | CH₃ | H | OC₃H₇-i | cyclo-propyl |
| A163 | CH₃ | H | cyclo-propyl | cyclo-propyl |
| A164 | CH₃ | H | CH₃ | CH₃ |
| A165 | CH₃ | H | C₂H₅ | CH₃ |
| A166 | CH₃ | H | SCH₃ | CH₃ |
| A167 | CH₃ | H | OCH₃ | CH₃ |
| A168 | CH₃ | H | CH₂OCH₃ | cyclo-propyl |
| A169 | CH₃ | H | OC₂H₅ | CH₃ |
| A170 | CH₃ | H | OCH₃ | cyclo-propyl |
| A171 | CH₃ | H | C₂H₅ | OC₂H₅ |
| A172 | CH₃ | H | C₂H₅ | cyclo-propyl |

Die vorstehend aufgeführten Wirkstoffe sind z.B. bekannt aus "The Pesticide Manual", 13. Auflage (2003), The British Crop Protection Council oder den nach den einzelnen Wirkstoffen aufgeführten Literaturstellen.

Soweit in dieser Beschreibung der Begriff Acylrest verwendet wird, bedeutet dieser den Rest einer organischen Säure, der formal durch Abspaltung einer OH-Gruppe aus der organischen Säure entsteht, z.B. der Rest einer Carbonsäure und Reste davon abgeleiteter Säuren wie der Thiocarbonsäure, gegebenenfalls N-substituierten Iminocarbonsäuren oder die Reste von Kohlensäuremonoestern, gegebenenfalls N-substituierter Carbaminsäuren, Sulfonsäuren, Sulfinsäuren, Phosphonsäuren, Phosphinsäuren.

Ein Acylrest ist bevorzugt Formyl oder Acyl aus der Gruppe CO-R^{z}, CS-R^{z}, CO-OR^{z}, CS-OR^{z}, CS-SR^{z}, SOR^{z} oder SO₂R^{z}, wobei R^{z} jeweils einen C₁-C₁₀-Kohlenwasserstoffrest wie C₁-C₁₀-Alkyl oder C₆-C₁₀-Aryl bedeutet, der unsubstituiert oder substituiert ist, z.B. durch einen oder mehrere Substituenten aus der Gruppe Halogen wie F, Cl, Br, I, Alkoxy, Haloalkoxy, Hydroxy, Amino, Nitro, Cyano oder Alkylthio, oder R^{z} bedeutet Aminocarbonyl oder Aminosulfonyl, wobei die beiden letztgenannten Reste unsubstituiert, N-monosubstituiert oder N,N-disubstituiert sind, z.B. durch Substituenten aus der Gruppe Alkyl oder Aryl. Acyl bedeutet beispielsweise Formyl, Halogenalkylcarbonyl, Alkylcarbonyl wie (C₁-C₄)Alkylcarbonyl, Phenylcarbonyl, wobei der Phenylring substituiert sein kann, oder Alkyloxycarbonyl, wie (C₁-C₄) Alkyloxycarbonyl, Phenyloxycarbonyl, Benzyloxycarbonyl, Alkylsulfonyl, wie (C₁-C₄) Alkylsulfonyl, Alkylsulfinyl, wie C₁-C₄(Alkylsulfinyl), N-Alkyl-1-iminoalkyl, wie N-(C₁-C₄)-1-imino-(C₁-C₄)alkyl und andere Reste von organischen Säuren.

Ein Kohlenwasserstoffrest bedeutet ein geradkettiger, verzweigter oder cyclischer und gesättigter oder ungesättigter aliphatischer oder aromatischer Kohlenwasserstoffrest, z.B. Alkyl, Alkenyl, Alkinyl, Cycloalkyl, Cycloalkenyl oder Aryl. Ein Kohlenwasserstoffrest weist bevorzugt 1 bis 40 C-Atome, vorzugsweise 1 bis 30 C-Atome auf; besonders bevorzugt bedeutet ein Kohlenwasserstoffrest Alkyl, Alkenyl oder Alkinyl mit bis zu 12 C-Atomen oder Cycloalkyl mit 3, 4, 5, 6 oder 7 Ringatomen oder Phenyl.

Aryl bedeutet ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl.

Ein heterocyclischer Rest oder Ring (Heterocyclyl) kann gesättigt, ungesättigt oder heteroaromatisch und unsubstituiert oder substituiert sein; er enthält vorzugsweise ein oder mehrere Heteroatome im Ring, vorzugsweise aus der Gruppe N, O und S; vorzugsweise ist er ein aliphatischer Heterocyclylrest mit 3 bis 7 Ringatomen oder ein heteroaromatischer Rest mit 5 oder 6 Ringatomen und enthält 1, 2 oder 3 Heteroatome. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält, beispielsweise Pyridyl, Pyrimidinyl, Pyridazinyl, Pyrazinyl, Triazinyl, Thienyl, Thiazolyl, Oxazolyl, Furyl, Pyrrolyl, Pyrazolyl und Imidazolyl, oder ist ein partiell oder vollständig hydrierter Rest wie Oxiranyl, Oxetanyl, Pyrrolidyl, Piperidyl, Piperazinyl, Triazolyl, Dioxolanyl, Morpholinyl, Tetrahydrofuryl. Bevorzugt sind Pyrimidinyl und Triazinyl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Substituierte Reste, wie substituierte Kohlenwasserstoffreste, z.B. substituiertes Alkyl, Alkenyl, Alkinyl, Aryl, Phenyl und Benzyl, oder substituiertes Heterocyclyl oder Heteroaryl, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, und Alkylsulfinyl, Haloalkylsulfinyl, Alkylsulfonyl, Haloalkylsulfonyl und, im Falle cyclischer Reste, auch Alkyl und Haloalkyl sowie den genannten gesättigten kohlenwasserstoffhaltigen Resten entsprechende ungesättigte aliphatische Reste, wie Alkenyl, Alkinyl, Alkenyloxy, Alkinyloxy etc. bedeuten. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy und Chlor.

Gegebenenfalls substituiertes Phenyl ist vorzugsweise Phenyl, das unsubstituiert oder ein- oder mehrfach, vorzugsweise bis zu dreifach durch gleiche oder verschiedene Reste, vorzugsweise aus der Gruppe Halogen, (C₁-C₄)Alkyl, (C₁-C₄)Alkoxy, (C₁-C₄)Halogenalkyl, (C₁-C₄)Halogenalkoxy und Nitro substituiert ist, z.B. o-, m- und p-Tolyl, Dimethylphenyl, 2-, 3- und 4-Chlorphenyl, 2-, 3- und 4-Trifluor- und -Trichlorphenyl, 2,4-, 3,5-, 2,5- und 2,3-Dichlorphenyl, o-, m- und p-Methoxyphenyl.

Cycloalkyl bedeutet ein carbocyclisches, gesättigtes Ringsystem vorzugsweise mit 3-6 C-Atomen, z.B. Cyclopropyl, Cyclobutyl, Cyclopentyl oder Cyclohexyl.

Die kohlenstoffhaltigen Reste wie Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.

Halogen bedeutet beispielsweise Fluor, Chlor, Brom oder lod. Haloalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl, CCl₃, CHCl₂, CH₂CH₂Cl⁻, Haloalkoxy ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Besonders bevorzugte Sulfonamide sind Sulfonylharnstoffe wie Amidosulfuron, Ethoxysulfuron, lodosulfuron-methyl, Mesosulfuron-methyl, Foramsulfuron, Propoxycarbazone.

In dem erfindungsgemäßen Verfahren können eines oder mehrere Sulfonamide, vorzugsweise ein Sulfonamid, eingesetzt werden. Die Sulfonamide sind bevorzugt Neutralverbindungen, die insbesondere zu Monosalzen umgesetzt werden, es können aber auch Sulfonamid-Monosalze eingesetzt und z.B. zu Disalzen umgesetzt werden. Bevorzugt wird ein Sulfonamid eingesetzt, welches als Neutralverbindung vorliegt.

Bei den aus den vorstehend genannten Sulfonamiden erhältlichen Sulfonamidsalzen handelt es sich vorzugsweise um Mono-Salze, insbesondere solche bei denen der Wasserstoff der -SO₂-NH-Gruppe des Sulfonamids durch ein für die Landwirtschaft geeignetes Kation ersetzt ist, z.B. um Metallsalze oder um Ammoniumsalze. Bevorzugte Sulfonamidsalze sind Metallsalze wie Alkalimetallsalze (z.B. Natrium- oder Kaliumsalze) und Erdalkalimetallsalze (z.B. Calcium- oder Magnesiumsalze). Besonders bevorzugte Sulfonamidsalze sind Natriumsalze und Kaliumsalze. Ganz besonders bevorzugt sind die Natriumsalze der Sulfonamide (A1), (A2), (A3), (A4), (A5), (A6), (A7), (A8), (A9), (A10), (A11), (A12), (A13), (A14), (A15), (A16), (A17), (A18), (A19), (A20), (A21), (A22), (A23), (A24), (A25), (A26), (A27), (A28), (A29), (A30), (A31), (A32), (A33), (A34), (A35), (A36), (A37), (A38), (A39), (A40), (A41), (A42), (A43), (A44), (A45), (A46), (A47), (A48), (A49), (A50), (A51), (A52), (A53), (A54), (A55), (A56), (A57), (A58), (A59), (A60), (A61), (A62), (A63), (A64), (A65), (A66), (A67), (A68), (A69), (A70), (A71), (A72), (A73), (A74), (A75), (A76), (A77), (A78), (A79), (A80), (A81), (A82), (A83), (A84), (A85), (A86), (A87), (A88), (A89), (A90), (A91), (A92), (A93), (A94), (A95), (A96), (A97), (A98), (A99), (A100), (A101), (A102), (A103), (A104), (A105), (A106), (A107), (A108), (A109), (A110), (A111), (A112), (A113), (A114), (A115), (A116), (A117), (A118), (A119), (A120), (A121), (A122), (A123), (A124), (A125), (A126), (A127), (A128), (A129), (A130), (A131), (A132), (A133), (A134), (A135), (A136), (A137), (A138), (A139), (A140), (A141), (A142), (A143), (A144), (A145), (A146), (A147), (A148), (A149), (A150), (A151), (A152), (A153), (A154), (A155), (A156), (A157), (A158), (A159), (A160), (A161), (A162), (A163), (A164), (A165), (A166), (A167), (A168), (A169), (A170), (A171), (A172) und die Kaliumsalze der Sulfonamide (A1), (A2), (A3), (A4), (A5), (A6), (A7), (A8), (A9), (A10), (A11), (A12), (A13), (A14), (A15), (A16), (A17), (A18), (A19), (A20), (A21), (A22), (A23), (A24), (A25), (A26), (A27), (A28), (A29), (A30), (A31), (A32), (A33), (A34), (A35), (A36), (A37), (A38), (A39), (A40), (A41), (A42), (A43), (A44), (A45), (A46), (A47), (A48), (A49), (A50), (A51), (A52), (A53), (A54), (A55), (A56), (A57), (A58), (A59), (A60), (A61), (A62), (A63), (A64), (A65), (A66), (A67), (A68), (A69), (A70), (A71), (A72), (A73), (A74), (A75), (A76), (A77), (A78), (A79), (A80), (A81), (A82), (A83), (A84), (A85), (A86), (A87), (A88), (A89), (A90), (A91), (A92), (A93), (A94), (A95), (A96), (A97), (A98), (A99), (A100), (A101), (A102), (A103), (A104), (A105), (A106), (A107), (A108), (A109), (A110), (A111), (A112), (A113), (A114), (A115), (A116), (A117), (A118), (A119), (A120), (A121), (A122), (A123), (A124), (A125), (A126), (A127), (A128), (A129), (A130), (A131), (A132), (A133), (A134), (A135), (A136), (A137), (A138), (A139), (A140), (A141), (A142), (A143), (A144), (A145), (A146), (A147), (A148), (A149), (A150), (A151), (A152), (A153), (A154), (A155), (A156), (A157), (A158), (A159), (A160), (A161), (A162), (A163), (A164), (A165), (A166), (A167), (A168), (A169), (A170), (A171), (A172).

Als Basen eignen sich insbesondere Verbindungen, bei denen der pKs-Wert der korrespondierenden Säure höher ist als der des eingesetzten Sulfonamids. Der pKs-Wert der korrespondierenden Säure kann auch niedriger als der des eingesetzten Sulfonamids sein, z.B. wenn die zu der zugesetzten Base korrespondierende Säure während der Deprotonierungsreaktion effizient aus dem Gleichgewicht entfernt werden kann und dadurch ein erhöhter Umsatz erzielt werden kann.

Geeignete Basen sind z.B. anorganische und organische Basen. Geeignete Basen können ein oder mehrere Protonen aufnehmen und bilden kationische Gegenionen zu Sulfonamidanionen. Bei den Basen kann es sich um Monomere, oligomere oder polymere Verbindungen handeln.

Anorganische Basen sind z.B. Alkalihydroxide (z.B. Natriumhydroxid oder Kaliumhydroxid) oder Erdalkalihydroxide, Alkalicarbonate (z.B. Natriumcarbonat oder Kaliumcarbonat) oder Erdalkalicarbonate, Alkalihydrogencarbonate (z.B. Natriumhydrogencarbonat oder Kaliumhydrogencarbonat) oder Erdalkalihydrogencarbonate.

Organische Basen sind z.B. kohlenwasserstoffhaltige Basen mit negativer Ladung auf einem C, O oder N-Atom (oder auch delokalisierte Ladung über mehrere Atome), es handelt sich dabei in der Regel um Salze von CH-aciden organischen Säuren wie Malonaten (z.B. Dialkylmalonaten), von OH-aciden Verbindungen wie Alkoholen, Phenolen, Enolaten, oder von NH-aciden Verbindungen wie Amiden. Die organischen Basen können auch polymerer Natur sein.

Beispiele bevorzugter organischer Basen sind Alkoholate, insbesondere monofunktionale Alkoholate, z.B. monofunktionale Alkalialkoholate wie RONa oder ROK, wobei R ein (C₁-C₁₆)Alkylrest wie Me, Et, n-Pr, i-Pr, n-Bu, i-Bu, s-Bu oder t-Bu ist,
CH-acide organische Säuren wie Malonate (z.B. Di-O-(C₁-C₁₆)alkylmalonate wie Di-O-methylmalonat oder Di-O-ethylmalonat),
NH-acide Säure wie Amide, z.B. R'-CO-NHR", worin R' und R" gleich oder voneinander verschieden H oder ein substituierter oder unsubstituierter Kohlenwasserstoffrest wie C₁-C₁₆-Alkyl sind,
polymere Basen, die z.B. erhalten werden indem Polymere, die eine Amidfunktion enthalten, mit Basen z.B. Hydroxiden wie NaOH oder KOH oder Alkoholaten wie Alkalialkoholaten (z.B. Natriummethanolat oder Kaliummethanolat) in polymere Basen überführt werden, z.B. Polymere der Formel (A) in polymere Basen der Formel (B) überführt werden 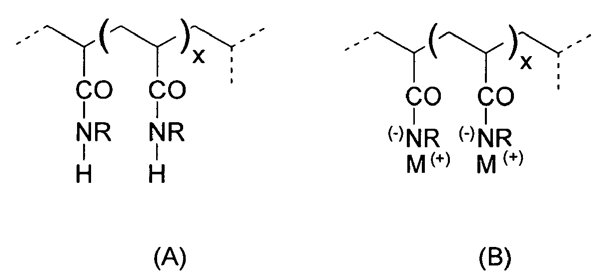 , worin
- R: H, C₁-C₁₀-Alkyl oder C₆-C₁₄-Aryl ist,
- M⁽⁺⁾: ein Kation, bevorzugt ein Alkalimetallkation wie Na oder K ist, und
- x: eine ganze Zahl von 10 bis 2 000 000 ist.

Im allgemeinen lassen sich die in Schritt a) entstehenden korrespondierenden Säuren der eingesetzten Basen (z.B. entstehendes Wasser, Alkohole oder CO₂) einfach entfernen, z.B. nach oder gegebenenfalls auch während der Reaktion. Beispielsweise können sie über die Gasphase (z.B. durch Abdestillieren) aus dem Reaktionsgemisch entfernt werden oder während eines optionalen Reinigungsschritts oder während des Trocknungsschritts b). Ausnahmsweise können sie auch im Produkt verbleiben, z.B. wenn die korrespondierende Säure Bestandteil einer daraus herzustellenden Wirkstoffformulierung ist.

Die Basen werden in der Regel stöchiometrisch zu den verwendeten Sulfonamiden eingesetzt, es kann jedoch auch Unter- oder Überdosierung vorgenommen werden, insbesondere im Bereich von +/- 10%, bezogen auf die Äquivalente eingesetzten Sulfonamids. Es können auch Basengemische zum Einsatz kommen, z.B. Gemische aus den oben genannten Basen.

Als Lösungsmittel eignen sich reine Lösungsmittel oder auch Gemische aus verschiedenen Lösungsmittelkomponenten. Geeignete Lösungsmittel zeichnen sich dadurch aus, dass sie sich gegenüber dem eingesetzten Sulfonamid und dessen erhaltenen Salzen unter den gewählten Reaktionsbedingungen im wesentlichen chemisch inert verhalten.

Zudem erlauben sie im allgemeinen eine Reaktionsführung, bei der der Anteil der Sulfonamide am Reaktionsgemisch mehr als 10 Gew.-%, vorzugsweise mehr als 20 Gew.-% beträgt. Weiterhin lassen sich geeignete Lösungsmittel in der Regel einfach entfernen, zum Beispiel während der Trocknung, z.B. durch Sprühtrocknung oder Wirbelschichttrocknung.

Als geeignete Lösungsmittel kommen z.B. in Frage Wasser oder organische Lösungsmittel wie unpolare organische Lösungsmittel oder polare organische Lösungsmittel (z.B. polar aprotische oder polar protische). Beispiele für organische Lösungsmittel sind:
1) unpolare organische Lösungsmittel wie
1a) Kohlenwasserstoffe, die unsubstituiert oder mit (C₁-C₁₀)Kohlenwasserstoffresten wie (C₁-C₁₀)Alkyl substituiert sein können, z.B. aromatische Kohlenwasserstoffe, z.B.
   1. ein- oder mehrfach alkyl-substituierte Benzole wie Toluol, Xylole, Mesitylen, Ethylbenzol, oder
   2. ein- oder mehrfach alkyl-substituierte Naphthaline wie 1-Methylnaphthalin, 2-Methylnaphthalin oder Dimethylnaphthalin, oder
   3. andere vom Benzol abgeleitete aromatische Kohlenwasserstoffe, wie Indan oder Tetralin^{®} , oder
   4. Gemische hieraus,
      aliphatische Kohlenwasserstoffe, z.B.
   5. lineare oder verzweigte Aliphaten, z.B. der allgemeinen Formel CₙH₂ₙ₊₂, wie Pentan, Hexan, Octan, 2-Methylbutan oder 2,2,4-Trimethylpentan, oder
   6. cyclische, gegebenenfalls alkyl-substituierte Aliphaten, wie Cyclohexan oder Methylcyclopentan, oder
   7. Gemische hieraus, wie Lösungsmittel der Exxsol^{®}D-Reihe, Isopar^{®}-Reihe oder Bayol^{®}-Reihe z.B. Bayol^{®}82 (ExxonMobil Chemicals) oder der Isane^{®}IP-Reihe oder Hydroseal^{®}G-Reihe (TotalFinaElf),
   Gemische von aromatischen und aliphatischen Kohlenwasserstoffen, wie Lösungsmittel der Solvesso^{®}-Reihe z.B. Solvesso^{®}100, Solvesso^{®}150 oder Solvesso^{®}200 (ExxonMobil Chemicals), der Solvarex^{®}/Solvaro^{®}-Reihe (TotalFinaElf) oder der Caromax^{®}-Reihe z.B. Caromax^{®}28 (Petrochem Carless), oder
1b) Öle natürlicher Herkunft oder synthetischer Herkunft, z.B. Pflanzenöle wie Maiskeimöl oder Rapsöl und deren Umesterungsprodukte wie Rapsölmethylester oder Rapsölethylester;
2) polare organische Lösungsmittel wie
2a) polar aprotische Lösungsmittel wie Ether, Ester von C₁-C₉-Alkansäuren, die mono-, di- oder polyfunktional sein können, wie deren Mono-, Di- oder Triester, z.B. mit C₁-C₁₈-Alkylalkoholen, halogenierte Kohlenwasserstoffe wie halogenierte aromatische und halogenierte aliphatische Kohlenwasserstoffe, z.B. Chlorbenzol oder Methylenchlorid, Phophorsäureester, Amide, Nitrile oder Sulfone, z.B. Diisobutyladipat, Rhodiasolv^{®} RPDE (Rhodia), Cyclohexanon, Jeffsol^{®}PC (Propylencarbonat, Huntsman), γ-Butyrolacton, N-Methylpyrrolidon, Dimethylsulfoxid, Acetonitril, Tributylphosphatam oder Hostarex^{®}PO-Reihe (Clariant), oder
2b) polar protische Lösungsmittel wie Alkohole oder Enole. Die Alkohole und Enole können ein- oder mehrwertig, aliphatisch, cycloaliphatisch, aromatisch oder heteroaromatisch sein. Bevorzugt sind ein- oder zweiwertige aliphatische Alkohole, z.B. Ethylenglykol oder Propylenglykol oder C₁-C₆-Alkylalkohole wie Methanol, Ethanol, n-Propanol, iso-Propanol, n-Butanol, iso-Butanol, sec.-Butanol, tert.-Butanol.

Die genannten Lösungsmittel können allein oder im Gemisch verwendet werden.

Bevorzugt sind als Lösungsmittel Wasser und polare organische Lösungsmittel wie polar aprotische oder polar protische Lösungsmittel, oder deren Gemische. Besonders bevorzugt sind als Lösungsmittel Wasser und Gemische von Wasser mit polaren organischen Lösungsmitteln wie Alkoholen z.B. C₁-C₆-Alkylalkoholen wie Methanol, Ethanol, n-Propanol, iso-Propanol, n-Butanol, iso-Butanol, sec.-Butanol, tert.-Butanol.

Die Umsetzung in Schritt a) erfolgt in der Regel unter kräftiger Durchmischung. Das Reaktionsgemisch kann homogen oder heterogen (d.h. eine Komponente ist teilweise oder vollständig im Lösungsmittel ungelöst) sein, oder sich auch während der Reaktionsführung von heterogen zu homogen oder umgekehrt ändern. Geeignete Geräte für die Durchmischung sind z.B. Rührer wie Rotor-Stator-Mischer (z.B. Ultraturrax), Mühlen (z.B. Perlmühlen) oder eine Kombination aus mehreren Geräten, z.B. Rührer und Mühle.

Falls nach der Umsetzung a) das Reaktionsgemisch als heterogenes Gemisch vorliegt, ist es bevorzugt das Gemisch intensiv zu homogenisieren (z.B. mittels eines Rotor-Stator-Mischers oder einer Perl-Mühle), als Vorbereitung für den Trocknungsschritt b) (z.B. die Sprühtrocknung oder Wirbelschichttrocknung).

Das in Schritt a) erhaltene Reaktionsgemisch kann optional einem Reinigungsschritt unterworfen werden. Beispiele hierfür sind Filtration (z.B. um kleine Partikel ungelöster Stoffe oder gebildeter Verunreinigungen abzutrennen) oder Extraktion (z.B. um Neben-Komponenten oder Verunreinigungen aus dem Reaktionsgemisch zu entfernen). Als Lösungsmittel für eine Extraktion kommen insbesondere Lösungsmittel in Frage, die mit dem erhaltenen Reaktionsgemisch nicht mischbar sind.

Es können dem Reaktionsgemisch auch kleine Mengen an einem oder mehreren Hilfsstoffen wie Entschäumer oder Tenside (z.B. Netzmittel), zugesetzt werden, z.B. um die Umsetzung a) zu beschleunigen oder Schaumbildung zu vermeiden oder zu unterdrücken. Der Gehalt an solchen Hilfsstoffen ist im allgemeinen ≤ 5 Gew.-%, bevorzugt ≤ 1 Gew.-%, jeweils bezogen auf das Gesamtreaktionsgemisch. Die Hilfsstoffe sind dem Fachmann bekannt, z.B. aus Pestizide Formulations, Marcel Dekker, N.Y., 1973; McCutcheon's "Detergents and Emulsifiers Annual", MC Publ. Corp., Ridgewood M.J. Sisley and Wood, "Encyclopedia of Surface Active Agents", Chem. Publ. Co. lnc., N.Y. 1964; Schönfeldt, "Ganzflächenaktive Äthylenoxidaddukte", Wiss. Verlagsgesellschaft, Stuttgart 1976.

Die Reaktionstemperatur für die Umsetzung a) liegt im allgemeinen zwischen 0°C und 100°C, vorzugsweise zwischen 10°C und 100°C, besonders bevorzugt zwischen 10°C und 90°C. Die Temperatur kann sich auch während der Reaktionsführung ändern, z.B. kann es günstig sein die Reaktionstemperatur mit zunehmender Reaktionsdauer zu erhöhen oder zu erniedrigen.

In Schritt b) werden durch Versprühen des in Schritt a) oder in einem optional nachgeschalteten Reinigungsschritt und/oder Homogenisierungsschritt erhaltenen Produkts in einer Trocknungsvorrichtung, z.B. in einer Sprühtrocknungsvorrichtung oder in einer Granulierungsvorrichtung, flüchtige Komponenten wie das Lösungsmittel von dem Sulfonamidsalz abgetrennt (Trocknung). Die Trocknung durch Versprühen kann nach an sich bekannten Verfahren durchgeführt werden, z.B. durch Sprühtrocknung oder durch Wirbelschichttrocknung. Das Sulfonamidsalz fällt nach der Trocknung b) in fester Form an.

Trocknungsvorrichtungen, in welchen das erhaltene Produkt versprüht werden kann, sind in dem Fachmann bekannt (siehe z.B. H. Mollet, A. Grubenmann, Formulierungstechnik, Verlag Wiley - VCH, 2000, Kapitel 6.2.5, 6.2.7 und 6.2.8). Beispiele solcher Trocknungsvorrichtungen sind z.B. in EP-A-0757891, EP-A-611 593, EP-A-821 618, US 5,883,047 und WO 98/42192 beschrieben.

Zur Trocknung können verschiedene dem Fachmann geläufige Trocknungsverfahren verwendet werden (siehe z.B. H. Mollet, A. Grubenmann, Formulierungstechnik, Verlag Wiley - VCH, 2000, Kapitel 6.2.5, 6.2.7 und 6.2.8), von denen nachfolgend einige exemplarisch beschrieben sind.

Die Trocknung kann z.B. mittels Sprühtrocknung erfolgen. Dabei wird das Produkt durch Düsen in eine genügend lange, vertikale Fallstrecke hineinversprüht. Die entstandenen Tröpfchen trocknen während des Falls ab - bevorzugt durch Verwendung eines warmen (in der Regel 50 bis 300°C, vorzugsweise 50 - 150°C, besonders 100 bis 140°C) Prozessgasstroms (z.B. Luft oder Stickstoff) - und fallen am Boden der Apparatur als feines Granulat an. Apparate hierzu werden z.B. von der Firma Niro hergestellt, z.B. der Typ Niro Atomizer.

Den Ausführungsformen der Sprühtrocknung ist gemeinsam, dass das flüssige Gemisch zunächst in eine Trocknungsvorrichtung, wie eine Trocknungskammer eingedüst und dabei in einzelne Tröpfchen zerrissen ("atomatisiert") wird. Das Eindüsen kann mit und ohne Unterstützung durch Druckgas (z.B. Luft oder Stickstoff) geschehen. Bei den Düsen kann es sich um einzelne Bauteile oder eine Gruppe aus mehreren Düsen handeln. Die Düsen können für Ein-, Zwei- oder Mehrfachstoffverdüsung ausgelegt sein (z.B. WO 2004/073403 oder K. Mertens "Spray Drying" Handbook, 3rd Ed. 1979, G. Godwin Ltd., London)

Die Tröpfchen durchfliegen eine einfache Fallstrecke, wobei sie ihren Feuchtigkeitsgehalt an den Prozessgasstrom (z.B. Luft oder Stickstoff) abgeben. Die festen Bestandteile werden dann als Staub oder aber als Granulat durch geeignete Vorrichtungen aufgefangen (z.B. Siebboden, Zyklon-Abscheider). Die gewonnenen Festpartikel können entweder kontinuierlich oder diskontinuierlich ausgetragen werden.

Eine andere Trocknungsform ist die Wirbelschichttrocknung. Dabei wird das Produkt durch Düsen in eine von einem Prozessgasstrom (z.B. Luft oder Stickstoff) entgegen der Schwerkraft kontinuierlich durchströmte - anfangs bevorzugt leere - Kammer (Trocknungskammer) eingesprüht. Durch den Prozessgasstrom trocknen die entstandenen Tröpfchen ab und werden in dauerndem Schwebezustand (Wirbelbett) gehalten. Apparate zur Wirbelschichttrocknung werden z.B. von der Firma Aeromatic hergestellt, für Technikumszwecke etwa der Typ MP-1.

Bei der Wirbelschichttrocknung werden die Tröpfchen durch einen in Gleich- oder Gegenrichtung eingeblasenen Gasstrom (z.B. Luft oder Stickstoff) gegen die Schwerkraft in Schwebe gehalten und geben ihre Feuchtigkeit an diesen Gasstrom, der ggf. erwärmt ist, ab. Die verbliebenen Fest-Partikel oder Granulate können entweder kontinuierlich oder diskontinuierlich ausgetragen werden.

Die Ausführungsformen der Wirbelschichttrocknung lassen sich durch folgende Angaben näher beschreiben, die im allgemeinen von Typ und Kapazität Wirbelschichttrocknungsanlage abhängen:
• Die Größe der Tröpfchen (nach Verdüsung) kann innerhalb eines weiten Bereiches variiert werden. Die Mehrzahl der Tröpfchendurchmesser schwankt zwischen 0,05 und 5 mm, bevorzugt zwischen 0,05 und 2 mm.
• Die Sprührate beträgt in der Regel 1 bis 1000 kg/h, bevorzugt 10 bis 500 kg/h.
• Die Granuliertemperatur beträgt in der Regel 20 bis 100°C, vorzugsweise 40 bis 80°C.
• Die Temperatur des Prozessgases beträgt in der Regel 50 bis 300°C, vorzugsweise 50 - 150°C, besonders 100 bis 140°C.
• Die Gasströme schwanken in der Regel zwischen 1 und 100.000 norm-Kubikmeter/h, vorzugsweise zwischen 10 und 80.000 norm-Kubikmeter/h, besonders zwischen 10 und 50.000 Norm-Kubikmeter/h.
• Die Wirbelschichttrocknungen können kontinuierlich oder diskontinuierlich gefahren werden.
• Die Wirbelschichttrocknungen können auch nach dem Vorlageverfahren erfolgen, d.h. es werden kleine Partikel des zu gewinnenden Sulfonamidsalzes vorgelegt, auf die dann flüssigen Reaktionsgemische enthaltend Lösungsmittel und Sulfonamidsalz aufgesprüht werden.

### Beispiele für Trocknungsvorrichtungen sind Glatt GPCG5 (Firma Glatt), Aeromatik MP1 (Firma Aeromatik-Fielder AG, CH), Büchi 190 (Firma Büchi Labortechnik AG, CH).

Das erfindungsgemäße Verfahren zeichnet sich durch verschiedene Vorteile aus wie hoher Durchsatz, hoher Ausbeute, hohe Produktreinheit, definierte und reproduzierbare Partikelgrößenverteilung sowie reproduzierbare Restfeuchte.

Die nach dem erfindungsgemäßen Verfahren erhaltenen Sulfonamidsalze können - in der Regel ohne weiteren Aufarbeitungsschritt - vorteilhaft zur Herstellung fester oder flüssiger agrochemischer Formulierungen verwendet werden. Die erfindungsgemäß erhaltenen Sulfonamidsalze können dabei auf verschiedene Art formuliert werden, je nachdem welche biologischen und/oder chemisch-physikalischen Parameter vorgegeben sind. Als Formulierungstypen kommen beispielsweise in Frage: Spritzpulver (WP), wasserlösliche Pulver (SP), wasserlösliche Konzentrate (SL), emulgierbare Konzentrate (EC, siehe z.B. WO 00/56146, WO 01/82693), Emulsionen (EW), wie Öl-in-Wasser- und Wasser-in-Öl-Emulsionen, versprühbare Lösungen, Suspensionskonzentrate (SC), Ölsuspensionskonzentrate (OD) (siehe z.B. WO 01/30156 A1 ; WO 01/82693 A2; WO 04/054364, WO 05/011382), Suspensionskonzentrate auf Wasserbasis, ölmischbare Lösungen, Kapselsuspensionen (CS), Stäubemittel (DP), Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen und deren Herstellung sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London. Bevorzugte Formulierungstypen sind Spritzpulver (WP), Granulate (z.B. GR, WG oder SG), Ölsuspensionskonzentrate (OD) und emulgierbare Konzentrate (EC). Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen, von Sulfonamidsalzen A) verschiedenen, agrochemischen Wirkstoffen B) wie Insektiziden, Akariziden, Herbiziden, Fungiziden, Safenern, Düngemitteln wie Ammoniumsulfat, Ammoniumhydrogensulfat, Harnstoff oder Mischungen davon, und/oder Wachstumsregulatoren herstellen, z.B. in Form einer Fertigformulierung oder als Tankmix.

Daneben enthalten die genannten Wirkstofformulierungen gegebenenfalls übliche Hilfsstoffe wie Haft-, Netz-, Dispergier-, Emulgier-, Penetrations-, Konservierungs-, Frostschutz- und Lösungsmittel, Füll-, Träger- und Farbstoffe, Entschäumer, Stabilisatoren, Adjuvants wie Mineral- oder Pflanzenöle und deren Derivate, Verdunstungshemmer und den pH-Wert und die Viskosität beeinflussende Mittel.

Die Formulierungen lassen sich durch übliche Verfahren herstellen, z.B. durch Vermischen der Komponenten beispielsweise mit Hilfe von Rührern.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Zur Anwendung können die agrochemischen Wirkstoffformulierungen gegebenenfalls in üblicher Weise, z.B. zu Emulsionen, Suspensionen, Suspoemulsionen oder Lösungen verdünnt werden, z.B. mittels Wasser. Es kann vorteilhaft sein, erhaltenen Spritzbrühen weitere agrochemische Wirkstoffe (z.B. Tankmischpartner in Form entsprechender Formulierungen) und/oder zur Anwendung übliche Hilfs- und Zusatzstoffe, z.B. selbstemulgierende Öle wie Pflanzenöle oder Paraffinöle und/oder Düngemittel zuzugeben. Die Spritzbrühen können z.B. im Vorauflauf oder Nachauflauf appliziert werden, beispielsweise auf Pflanzen, Pflanzensamen oder die Fläche auf der die Pflanzen wachsen.

Die Spritzbrühen weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden gut erfaßt. Dabei können die Mittel z.B. im Vorsaat-, Vorauflauf- oder Nachauflaufverfahren ausgebracht werden. Im einzelnen seien beispielhaft einige Vertreter der mono- und dikotylen Unkrautflora genannt, die kontrolliert werden können, ohne daß durch die Nennung eine Beschränkung auf bestimmte Arten erfolgen soll.

Auf der Seite der monokotylen Unkrautarten werden z.B. Apera spica venti, Avena spp., Alopecurus spp., Brachiaria spp., Digitaria spp., Lolium spp., Echinochloa spp., Panicum spp., Phalaris spp., Poa spp., Setaria spp. sowie Bromus spp. wie Bromus catharticus, Bromus secalinus, Bromus erectus, Bromus tectorum und Bromus japonicus und Cyperusarten aus der annuellen Gruppe und auf seiten der perennierenden Spezies Agropyron, Cynodon, Imperata sowie Sorghum und auch ausdauernde Cyperusarten gut erfaßt.

Bei dikotylen Unkrautarten erstreckt sich das Wirkungsspektrum auf Arten wie z.B. Abutilon spp., Amaranthus spp., Chenopodium spp., Chrysanthemum spp., Galium spp. wie Galium aparine, lpomoea spp., Kochia spp., Lamium spp., Matricaria spp., Pharbitis spp., Polygonum spp.,Sida spp., Sinapis spp., Solanum spp., Stellaria spp., Veronica spp. und Viola spp., Xanthium spp., auf der annuellen Seite sowie Convolvulus, Cirsium, Rumex und Artemisia bei den perennierenden Unkräutern.

Unter den spezifischen Kulturbedingungen im Reis vorkommende Schadpflanzen wie z.B. Echinochloa, Sagittaria, Alisma, Eleocharis, Scirpus und Cyperus werden von den erfindungsgemäßen Mitteln ebenfalls hervorragend bekämpft.

Obgleich die Spritzbrühen eine ausgezeichnete herbizide Aktivität gegenüber mono- und dikotylen Unkräutern aufweisen, werden Kulturpflanzen wirtschaftlich bedeutender Kulturen z.B. zweikeimblättriger Kulturen wie Soja, Baumwolle, Raps, Zuckerrüben, oder Gramineen-Kulturen wie Weizen, Gerste, Roggen, Hafer, Hirse, Reis oder Mais, nur unwesentlich oder gar nicht geschädigt und eignen sich aus diesen Gründen sehr gut zur Bekämpfung von unerwünschtem Pflanzenwuchs, insbesondere zur selektiven Bekämpfung von unerwünschtem Pflanzenwuchs in landwirtschaftlichen Nutzpflanzungen oder in Zierpflanzungen.

Beispiel

### 20 g Foramsulfuron wurden in 75 g Wasser suspendiert, anschließend wurde unter kräftiger Durchmischung mit einem Ultraturrax Rotor-Stator-Mischer ein Äquivalent Natriumhydroxid bei Raumtemperatur zugesetzt.

Das resultierende Reaktionsgemisch wurde anschließend in einer Büchi 190 Sprühtrocknungsanlage versprüht, bei folgenden Verfahrensparametern:

| | |
|---|---|
| Eingangstemperatur (Prozessgas): | 120 - 130°C |
| Ausgangstemperatur (Prozessgas): | 65 - 75°C |
| Prozessgas: | Luft |

Das erhaltene Produkt fiel mit hoher Reinheit (> 95 %) und Ausbeute (> 95 %) an.

## Patentansprüche

1. Verfahren zur Herstellung von Sulfonamidsalzen, enthaltend die Schritte
a) Umsetzung von einem oder mehreren Sulfonamiden mit einer oder mehreren Basen in einem oder mehreren Lösungsmitteln zu Sulfonamidsalzen,
b) Trocknung des in Schritt a) erhaltenen Produkts durch Versprühen in einer Trocknungsvorrichtung.

2. Verfahren gemäß Anspruch 1, worin ein Sulfonamid umgesetzt wird, ausgewählt aus der Gruppe der Phenylsulfonamide, Heteroarylsulfonamide oder Sulfondiamide.

3. Verfahren gemäß Anspruch 1 oder 2, worin als Base eine anorganische und/oder organische Base verwendet wird.

4. Verfahren gemäß einem oder mehreren der Ansprüche 1 bis 3, worin die Sulfonamidsalze Metallsalze oder ein Ammoniumsalze sind.

5. Verfahren gemäß einem oder mehreren der Ansprüche 1 bis 4, worin als Lösungsmittel Wasser und/oder organische Lösungsmittel verwendet werden.

6. Verfahren gemäß einem oder mehreren der Ansprüche 1 bis 5, worin das in Schritt a) erhaltene Produkt einem Reinigungsschritt und/oder einem Homogenisierungsschritt unterzogen wird.

7. Verfahren gemäß einem oder mehreren der Ansprüche 1 bis 6, worin in Schritt b) das in Schritt a) erhaltene Produkt, gegebenenfalls nach einem Reinigungsschritt und/oder Homogenisierungsschritt, in einer Sprühtrocknungsvorrichtung oder in einer Granulierungsvorrichtung versprüht wird.

8. Verwendung einer Sprühtrocknungsvorrichtung oder einer Granulierungsvorrichtung zur Herstellung fester Sulfonamidsalze.

9. Verfahren zur Herstellung einer agrochemischen Formulierung von Sulfonamidsalzen, enthaltend die Schritte
a) Umsetzung von einem oder mehreren Sulfonamiden mit einer oder mehreren Basen in einem oder mehreren Lösungsmitteln zu Sulfonamidsalzen,
b) Trocknung des in Schritt a) erhaltenen Produkts durch Versprühen in einer Trocknungsvorrichtung,
c) Überführung der in Schritt b) erhaltenen Sulfonamidsalze in eine agrochemische Formulierung.
